# EUROPÄISCHE PATENTSCHRIFT

(11) **EP 2 666 490 B1**
(45) Veröffentlichungstag und Bekanntmachung des Hinweises auf die Patenterteilung: **06.07.2016**
(21) Anmeldenummer: 13003996.9
(22) Anmeldetag: 19.02.2010
(51) Int. Cl.: A61M 1/10

(54) **Vorrichtung zur intermittierenden Okklusion eines Blutgefäßes**
Implantable device for intermittent occlusion of a blood vessel
Dispositif implantable destiné à l'occlusion intermittente d'un vaisseau sanguin

(43) Veröffentlichungstag der Anmeldung: 27.11.2013
(62) Teilanmeldung aus: 10450024.4
(73) Patentinhaber: MIRACOR Medical Systems GmbH, 1060 Wien (AT)
(72) Erfinder: Mohl, Werner, A-2571 Altenmarkt-Thennenberg (AT)
(74) Vertreter: Keschmann, Marc

(56) Entgegenhaltungen:
- WO-A1-2005/120602
- WO-A1-2008/064387
- WO-A2-03/008018
- US-A- 4 934 996
- US-A- 4 969 470

## Beschreibung

Die Erfindung betrifft eine Vorrichtung zur intermittierenden Okklusion eines Blutgefäßes, insbesondere einer das Organsystem drainierenden Vene, umfassend ein zu intermittierender Okklusion ansteuerbares Okklusionsmittel, eine mit dem Okklusionsmittel verbundene Steuereinrichtung, wenigstens einen Sensor zur kontinuierlichen oder in Zeitabständen vorgenommenen Erfassung wenigstens eines physiologischen Messwerts und einen vom Sensor gespeisten Messwertspeicher zum Speichern einer Messwertreihe.

Arterielles Blut, das den Herzmuskel versorgt, kann durch gesundes Herzgewebe hindurch treten und es ernähren, hat jedoch Schwierigkeiten, das ischämische Gewebe zu erreichen. Dadurch wird die Versorgung des ischämischen Gewebes mit Nährstoffen sowie das Abführen von Abbauprodukten des Metabolismus von dem ischämischen Gewebe behindert. In diesem Zusammenhang ist bereits vorgeschlagen worden, das ischämische Gewebe durch retrograde Perfusion mit Blut zu versorgen. Die Retroinfusion von Blut in Koronarvenen spielt insbesondere im Bereich der Myokardprotektion während der Chirurgie am offenen Herzen eine wichtige Rolle. Ein typischer derartiger Eingriff ist beispielsweise die Ballondilatation einer arteriosklerotisch verengten Koronararterie. Bei dieser auch als perkutane transluminale Koronarangioplastie (PTCA) bekannten Methode wird ein Ballonkatheter unter Röntgenkontrolle in den Bereich der Stenose der Koronararterie geführt und die arteriosklerotische Plaque durch Aufblasen des am Ende des Katheters befindlichen Ballons komprimiert. Während der Dilatation des Ballons findet stromabwärts in der Arterie keine Versorgung des Gewebes mit sauerstoffhaltigem Blut statt, wobei sich bereits bei Dilatationen von länger als 30 Sekunden Dauer funktionelle Veränderungen im Ischämiegebiet des Myokards feststellen lassen. Entsprechende Probleme der Ischämieprotektion des Myokards stellen sich auch bei anderen Eingriffen zur Koronarvaskularisierung wie z.B. bei Atherektomie, Koronarendoprothesen und Laseranwendungen. Auch beim akuten Herzinfarkt besteht eine Unterversorgung eines Myokardbezirks.

Im Zusammenhang mit der kurzzeitigen Ischämieprotektion wird seit einiger Zeit eine Retroinfusion von arteriellem Blut oder anderen nutritiven Flüssigkeiten in eine Vene des betreffenden Ischämiegebietes des Myokards durchgeführt. Das Blut wird dabei über die entsprechende Vene in die nutritiven Kapillaren des Ischämiegebietes gepumpt und versorgt so das Myokard in dieser Region mit Sauerstoff und Substraten.

Eine Vorrichtung zur Retroinfusion von Koronarvenen ist aus der US 4,934,996 bekannt geworden, mit welcher eine druckgesteuerte intermittierende Korönarsinusokklusion vorgenommen werden kann. Die Vorrichtung umfasst eine Einrichtung zum Okkludieren des Sinus, wie z.B. einen aufblasbaren Ballonkatheter, eine Druckmesseinrichtung zum Messen des Flüssigkeitsdruckes innerhalb des Koronarsinus und ein Steuergerät, das Auslösesignale für die Okklusionseinrichtung erzeugt, um eine Okklusion auszulösen oder aufzuheben. Das Steuergerät ist hierbei derart ausgelegt, dass im Koronarsinus das Druckmaximum während jedes Herzschlages gemessen, ein Plateauwert der Druckmaxima aufeinander folgender Herzschläge rechnerisch abgeschätzt und die Okklusion des Koronarsinus auf Basis des Plateauwertes der Druckmaxima aufgehoben wird.

Das Okkludieren des Koronarsinus bewirkt einen Druckanstieg und in der Folge eine Retroperfusion von Blut über die entsprechende Vene in die nutritiven Kapillaren des Ischämiegebietes, sodass diese Gebiete mit Nährstoffen versorgt werden können. Bei Aufheben der Okklusion wird das retroperfundierte Blut ausgeschwemmt, wobei gleichzeitig die Abfallprodukte des Metabolismus abgeführt werden. Bei dem Verfahren gemäß der US 9,934,996 wird somit aufgrund der Messung des Druckmaximums im Koronarsinus während jedes Herzschlages rechnerisch eine systolische Druckkurve abgeschätzt, wobei die intermittierende Okklusion in Abhängigkeit von dem Plateauwert der systolischen Druckkurve gesteuert wird. Der Verlauf der abgeschätzten systolischen Druckkurve lässt auch einen Rückschluss auf die Leistungsfähigkeit des Herzens zu, wobei beispielsweise die Steigung der Kurve die Kontraktilität des Herzens wiedergibt. Die Steigung der Kurve hat naturgemäß auch einen Einfluss auf die Höhe des Plateauwertes, wobei bei einer flacheren Kurve ein niedriger Plateauwert erreicht wird, wobei das Plateau überdies im Vergleich zu einem gesunden Herzen nach einer längeren Zeitspanne nach Einleiten der Okklusion erreicht wird. Eine Verschiebung der Kurve ergibt sich auch, wenn ein Koronargefäß bei einer interventionellen Handlung wie PTCA oder Stenting temporär oder durch eine Komplikation auch für länger verschlossen wird, sodass die Druckkurve langsamer ansteigt und auch länger braucht, bis das Plateau erreicht wird.

Verfahren zur intermittierenden Okklusion eines Blutgefäßes und insbesondere des Koronarsinus sind auch aus der WO 03/008018 A2, WO 2005/120602 A1 und WO 2005/120601 A1 bekannt geworden. Die vorgenannten Dokumente zeigen jeweils Vorrichtungen zur intermittierenden Okklusion eines Blutgefäßes, die ein zur intermittierenden Okklusion ansteuerbares Okklusionsmittel, eine mit dem Okklusionsmittel verbundene Steuereinrichtung, wenigstens einen Sensor und einen vom Sensor gespeisten Messwertspeicher zum Speichern einer Messwertreihe umfassen. Die mit diesen Vorrichtungen durchgeführten Verfahren sind auch bei akutem Herzinfarkt anwendbar, wobei nachgewiesen wurde, dass durch periodisches Okkludieren des venösen Abstroms eine Verkleinerung des Infarktareals erreicht werden kann. Weitere Verfahren zur intermittierenden Okklusion eines Blutgefäßes sind aus der WO 2008/064387 und der US 4969470 bekannt geworden.

Es ist auch bereits vorgeschlagen worden, Blut aus den Koronarvenen gut durchbluteter Areale in die schlecht versorgten Gefäßbezirke umzuleiten.

Die bisher bekannt gewordenen Vorrichtungen eignen sich ausschließlich zur temporären Behandlung eines Patienten während eines operativen Eingriffes. Ein derartiger Eingriff ist in der Regel mit einem erheblichen Aufwand verbunden und erfordert nicht nur für das Platzieren der Okklusionsvorrichtung im zu okkludierenden Blutgefäß einen Arzt. Auch der Betrieb der Okklusionsvorrichtung erfordert die ständige Überwachung durch einen Arzt. Während der intermittierenden Okklusion müssen kritische Parameter überwacht werden, wie beispielsweise der Druck im okkludierten Blutgefäß, und es müssen physiologische Messwerte ständig dahingehend analysiert werden, ob aufgrund der Behandlung die gewünschte Verbesserung des Zustandes und insbesondere eine Erhöhung der Leistungsfähigkeit des Herzens eingetreten ist. Weiters muss aufgrund der Messwerte entschieden werden, wann die Behandlung, d.h. der Vorgang der intermittierenden Okklusion beendet werden kann.

Ein mit den aus dem Stand der Technik bekannt gewordenen Verfahren und Vorrichtungen zur intermittierenden Okklusion eines Blutgefäßes verbundener Nachteil liegt auch darin, dass die Behandlung meist erst dann vorgenommen werden kann, wenn der Patient ernsthafte Beschwerden hat, d.h. zu einem Zeitpunkt, zu welchem möglicherweise bereits irreversible Schäden eingetreten sind. Eine präventive Anwendung der intermittierenden Okklusion des Blutgefäßes bereits bei einer geringen Abweichung bestimmter physiologischer Parameter von den patientenspezifischen Sollwerten ist in der Regel nicht möglich.

Die vorliegende Erfindung zielt nun darauf ab, die aus dem Stand der Technik bekannt gewordene Vorrichtung zur intermittierenden Okklusion eines Blutgefäßes dahingehend zu verbessern, dass der Behandlungsaufwand verringert wird und auf eine ständige ärztliche Kontrolle während der Behandlung verzichtet werden kann.

Die Erfindung zielt weiters darauf ab, die Steuerung der intermittierenden Okklusion weitestgehend zu automatisieren, wobei die Steuerung derart erfolgen soll, dass ein optimales Behandlungsergebnis erzielt werden kann.

Die der vorliegenden zugrunde liegende Aufgabe wird erfindungsgemäß durch eine Vorrichtung zur intermittierenden Okklusion eines Blutgefäßes, insbesondere einer das Organsystem drainierenden Vene gelöst, umfassend
- ein zu intermittierender Okklusion ansteuerbares Okklusionsmittel,
- eine mit dem Okklusionsmittel verbundene Steuereinrichtung,
- wenigstens einen Sensor zur kontinuierlichen oder in Zeitabständen vorgenommenen Erfassung wenigstens eines physiologischen Messwerts und
- einen vom Sensor gespeisten Messwertspeicher zum Speichern einer Messwertreihe,
die sich dadurch auszeichnet, dass die Messwertreihe der Steuereinrichtung zugeführt ist, die zur rechnerischen, insbesondere statistischen Auswertung der Messwertreine ausgebildet, ist und mit der Okklusionseinrichtung zum Beginnen oder zur Beendigung, des Vorgangs der intermittierenden Okklusion, bei welchem das Blutgefäß abwechselnd okkludiert und freigegeben wird, in Abhängigkeit vom Ergebnis der Auswertung, zusammenwirkt.

Mit der erfindungsgemäßen Vorrichtung lässt sich ein Verfahren zur Behandlung des Herzens bzw. von Durchblutungsstörungen durchführen, welches das intermittierende Okkludieren eines Blutgefäßes, insbesondere einer das Organsystem drainierenden Vene umfasst, und bei welchem das Blutgefäß abwechselnd okkludiert und freigegeben wird. Das Verfahren zeichnet sich dadurch aus, dass die Größe wenigstens eines physiologischen Werts des Patienten in Zeitabständen oder kontinuierlich ermittelt wird, dass die jeweiligen Messwerte gespeichert werden und eine Messwertreihe erhalten wird, dass eine rechnerische, insbesondere statistische Auswertung der Messwertreihe vorgenommen wird und dass in Abhängigkeit vom Ergebnis der Auswertung die intermittierende Okklusion begonnen oder beendet wird.

Mit dem genannten Verfahren und der Vorrichtung wird ein völlig automatisierter Betrieb ermöglicht, wobei aufgrund der rechnerischen und insbesondere statistischen Auswertung der Messwertreihe der optimale. Zeitpunkt ermittelt werden kann, zu welchem die Behandlung, bei welcher das Blutgefäß intermittierend okkludiert wird, begonnen und wann die Behandlung beendet werden soll. Ein derartiger automatisierter Betrieb ist insbesondere erforderlich, wenn die Okklusionsvorrichtung dauerhaft im Blutgefäß implantiert ist, da in diesem Fall eine Einwirkung durch den Arzt in der Regel nicht mehr möglich ist. Es ist daher erforderlich, dass die Steuervorrichtung aufgrund der ermittelten Messwerte entscheidet, ob und wie lange eine Behandlung mit Hilfe der intermittierenden Okklusion vorgenommen werden soll. Zu diesem Zweck ist erfindungsgemäß eine rechnerische und insbesondere statistische Auswertung der Messwertreihe vorgesehen, wobei in Abhängigkeit vom Ergebnis der Auswertung der Vorgang der intermittierenden Okklusion begonnen oder beendet wird. Der Vorgang der intermittierenden Okklusion umfasst hierbei, wie bereits erwähnt, eine Abfolge von abwechselnden Okklusionsphasen, bei welchen das Blutgefäß okkludiert wird und Freigabephasen, bei welchen das Blutgefäß freigegeben wird.

Um eine möglichst präzise Bestimmung des Zeitpunkts des Beginns oder der Beendigung der Behandlung zu ermöglichen, ist gemäß einer bevorzugten Weiterbildung vorgesehen, dass die rechnerische Auswertung nach jeder Messwertermittlung vorgenommen wird. Die rechnerische Auswertung berücksichtigt somit immer den aktuellsten Messwert, sodass eine ständige Aktualisierung der Auswertungsergebnisse vorgenommen wird.

Die rechnerische Auswertung selbst kann auf verschiedenste Art und Weise vorgenommen werden. Gemäß einer ersten bevorzugten Verfahrensweise ist vorgesehen, dass die rechnerische Auswertung der Messwertreihe die Umrechnung der einzelnen Messwerte der Messwertreihe in abgeleitete Messwerte umfasst, wobei eine abgeleitete Messwertreihe erhalten wird. Die Umrechnung ist beispielsweise dann erforderlich, wenn der für die Auswertung heranzuziehende physiologische Wert nicht unmittelbar gemessen werden kann. Als messbare Größen kommen im Rahmen der vorliegenden Erfindung, bevorzugt der Blutdruck im, Bereich des Blutgefäßes, der Blutmassen- oder -volumenfluss im Bereich des Blutgefäßes, der elektrische Widerstand, die Leitfähigkeit, die elektrische Impedanz im Bereich des Blutgefäßes, insbesondere des Herzens und/oder der Lunge, das EKG und/oder ein metabolischer Parameter, wie z.B. die 02-Sättigung und/oder der Laktatgehalt bzw. der pH-Wert des Blutes, in Frage.

Gemäß einer weiteren bevorzugten Verfahrensweise ist vorgesehen, dass die rechnerische Auswertung der Messwertreihe die Ermittlung der Differenz der jeweils letzten zwei Messwerte der Messwertreihe oder der Differenz der jeweils letzten zwei abgeleiteten Messwerte der abgeleiteten Messwertreihe umfasst, wobei eine Differenzwertreihe erhalten wird. Die Auswertung kann in diesem Zusammenhang derart vorgenommen werden, dass bei Auftreten einer vorgegebenen Differenz die intermittierende Okklusion begonnen oder beendet wird. Ein derartiges Sprungverhalten des Messwertes kann ein Indikator für einen kritischen Zustand des Blutgefäßes oder des Herzens sein, sodass unmittelbar mit der Behandlung begonnen werden muss. Vereinzelte Sprünge der Messwerte können aber auch als Ausreißer betrachtet werden, die beispielsweise auf Fehler in der Messwertermittlung zurückzuführen sind. Um derartige Ausreißer bei der Auswertung unberücksichtigt zu lassen, kann die rechnerische Auswertung bevorzugt eine statistische Auswertung umfassen, bei welcher eine Trenderkennung vorgenommen wird und kurzfristige Ausreißer unterdrückt werden.

Die rechnerische Auswertung der Messwertreihe kann den Vergleich der einzelnen Messwerte mit einem vorgegebenen absoluten Grenzwert umfassen, um bei Überschreiten oder Unterschreiten des Grenzwertes einen unzulässigen bzw. kritischen Zustand des Blutgefäßes bzw. Herzens zu erfassen. Alternativ kann aber auch ein relativer Grenzwert von Interesse sein und die Ausbildung ist in diesem Zusammenhang bevorzugt derart weitergebildet, dass die rechnerische Auswertung der Messwertreihe die Ermittlung der kumulierten Differenzen der Differenzwertreihe umfasst. Die kumulierte Differenz gibt hierbei die Differenz des letzten Messwertes vom ersten Messwert der Messwertreihe wieder.

Gemäß einer weiteren bevorzugten Verfahrensweise ist vorgesehen, dass die rechnerische Auswertung der Messwertreihe die Ermittlung der Änderung der Messwerte der Messwertreihe oder der abgeleiteten Messwerte der abgeleiteten Messwertreihe je Zeiteinheit umfasst, wobei eine Ableitungsreihe erhalten wird. Die Änderung der Messwerte je Zeiteinheit gibt hierbei die Geschwindigkeit der Änderung wieder und entspricht somit der ersten Ableitung der Messwertkurve nach der Zeit.

Eine Reihe von Messwerten ändert sich mit dem Zyklus des Herzschlages, beispielsweise der Blutdruck, der Volumen- bzw. Massenfluss des Blutes oder dgl., wobei in der Regel für die Auswertung lediglich der jeweilige Maximal- oder Minimalwert während eines Herzschlags von Interesse ist. Eine weitere bevorzugte Weiterbildung sieht daher vor, dass die rechnerische Auswertung der Messwertreihe die Ermittlung von lokalen Maxima und/oder lokalen Minima der Werte der Messwertreihe, der umgewandelten Messwertreihe, der Differenzwertreihe und/oder der Ableitungsreihe umfasst und dass aus den lokalen Maxima bzw. Minima eine Extremwertreihe gebildet wird.

Darüber hinaus ist es häufig der Fall, dass ein Messwert bzw. die während der jeweiligen Herzschläge auftretenden lokalen Maxima und/oder lokalen Minima während der Okklusionsphase mit jedem Herzschlag steigen oder fallen. In diesem Zusammenhang kann es von Vorteil sein, lediglich den während einer Okklusionsphase auftretenden Maximalwert für die Auswertung heranzuziehen. Eine weitere bevorzugte Verfahrensweise sieht daher vor; dass als lokales Maximum bzw. Minimum das jeweils während einer Okklusion auftretende Maximum bzw. Minimum der genannten Werte gewählt wird.

Die Steuereinrichtung, in der die rechnerische Auswertung der Messwerte bzw. Messwertreihe vorgenommen wird, kann in unterschiedlicher Weise mit der Okklusionsvorrichtung zusammenwirken, und es kann dementsprechend der Zeitpunkt des Beginns bzw. der Beendigung der intermittierenden Okklusion auf verschiedene Art und Weise ermittelt werden. Gemäß einer bevorzugten Weiterbildung ist im Rahmen der vorliegenden Erfindung vorgesehen, dass die rechnerische Auswertung der Messwertreihe den Vergleich der Werte der Messwertreihe, der abgeleiteten Messwertreihe, der Differenzwertreihe, der Ableitungsreihe und/oder der Extremwertreihe und/oder der kumulierten Differenzen mit einem vorgegebenen Grenzwert umfasst, wobei die intermittierende Okklusion bei Erreichen des Grenzwerts begonnen wird. Im Ruhezustand, in welchem keine intermittierende Okklusion vorgenommen wird, wird somit bei der rechnerischen Auswertung der Messwerte jeweils überwacht, ob ein vorgegebener Grenzwert erreicht wird. Beispielsweise kann anhand von Messwerten die Kontraktilität des Herzens überwacht werden. Weiters kann anhand von charakteristischen Veränderungen des EKGs, beispielsweise anhand der sog. "ST-Hebung" eine Ischämie festgestellt werden. Wenn die Kontraktilität oder die "ST"-Hebung einen Grenzwert, der patientenabhängig vorgegeben sein kann, erreicht oder unterschreitet, wird die Okklusionsvorrichtung zur Durchführung einer intermittierenden Okklusion angesteuert. Der Beginn der intermittierenden Okklusion kann beispielsweise auch anhand der 02-Sättigung des Blutes oder anhand des pH-Wertes und insbesondere anhand des Laktatgehalts festgestellt werden.

Eine weitere Möglichkeit, den optimalen Zeitpunkt des Beginns der intermittierenden Okklusion zu ermitteln besteht darin, die elektrische Impedanz des Thorax oder von Bereichen des Thorax; wie z.B. der Lunge zu ermitteln. Die elektrische Impedanz ist indirekt proportional zum Flüssigkeitsgehalt des erfassten Bereichs, sodass anhand der elektrischen Impedanz beispielsweise eine Linksherzinsuffizienz, bei der sich Blut in der Lunge anstaut, detektiert werden kann. In diesem Zusammenhang wird auf die WO 2008/070818 A2 verwiesen.

Während der intermittierenden Okklusion werden die Messwerte, wie beschrieben, ebenfalls ausgewertet, wobei der optimale Zeitpunkt zur Beendigung der intermittierenden Okklusion festgestellt werden muss. In diesem Zusammenhang sieht eine bevorzugte Weiterbildung vor, dass die rechnerische Auswertung der Messwertreihe die Erkennung oder Abschätzung eines Plateauwerts der Werte der Messwertreihe, der abgeleiteten Messwertreihe, der Differenzwertreihe, der Ableitungsreihe und/oder der Extremwertreihe umfasst, wobei die intermittierende Okklusion beim Plateauwert oder bei einem vorbestimmten Prozentwert des Plateauwerts beendet wird. Das Erreichen eines Plateauwerts zeigt hierbei an, dass ein charakteristischer Messwert sich verändert und einen stabilen Endwert erreicht hat. Bei Erreichen eines derartigen "steady state" haben die ursprünglich unter einen kritischen Wert gesunkenen oder über einen kritischen Wert angestiegenen physiologischen Parameter wiederum einen stabilen Normalwert erreicht, sodass der Vorgang der intermittierenden Okklusion beendet werden kann.

Bei einer bevorzugten Weiterbildung wird beim Beginnen und/oder Beenden der intermittierenden Okklusion jeweils eine neue Messwertreihe begonnen, sodass der Auswertung nur die jeweils nach dem letzten Beginnen oder Beenden der intermittierenden Okklusion ermittelten Messwerts zugrunde gelegt werden können.

Die ermittelten Messwerte können aber nicht nur zur Ermittlung des Beginns und des Endes der intermittierenden Okklusion herangezogen werden, sondern können auch zur Steuerung der einzelnen Okklusions- und Freigabephasen während der intermittierenden Okklusion verwendet werden. In diesem Zusammenhang ist bevorzugt vorgesehen, dass die während der Okklusion des Blutgefäßes ermittelten Messwerte jeweils einer gesonderten rechnerischen Auswertung unterzogen werden und dass die einzelnen Okklusionsphasen der intermittierenden Okklusion in Abhängigkeit vom Ergebnis der Auswertung beendet werden. Entsprechendes gilt für die Freigabephasen, wobei hier bevorzugt vorgesehen ist, dass die während der Freigabe des Blutgefäßes ermittelten Messwerte jeweils einer gesonderten rechnerischen Auswertung unterzogen werden und dass die einzelnen Freigabephasen der intermittierenden Okklusion in Abhängigkeit vom Ergebnis der Auswertung beendet werden.

Die erfindungsgemäße Vorrichtung ist besonders vorteilhaft bei einer implantierten Okklusionsvorrichtung, wie sie im folgenden näher beschreiben wird. Die implantierbare Vorrichtung zur intermittierenden Okklusion eines Blutgefäßes, insbesondere einer das Organsystem drainierenden Vene, wie z.B. des Koronarsinus, umfasst
- ein zu intermittierender Okklusion ansteuerbares und im Blutgefäß positionierbares Okklusionsmittel,
- wenigstens einen Sensor zur kontinuierlichen oder in Zeit abständen erfolgenden Erfassung wenigstens eines physiologischen Messwerts,
- eine bevorzugt implantierbare Steuereinrichtung, welcher der wenigstens eine physiologische Messwert zugeführt ist und die mit dem Okklusionsmittel zur Steuerung der intermittierenden Okklusion in Abhängigkeit vom Messwert zusammenwirkt, und
- ein mit dem Blutgefäß in Wirkverbindung bringbares, vom Okklusionsmittel gesondertes implantierbares Verankerungsmittel zum Positionieren des Okklusionsmittels relativ zum Blutgefäß. Das Okklusionsmittel ist zum Positionieren des selben relativ zum Blutgefäß insbesondere am Verankerungsmittel festgelegt.

Die implantierbare Vorrichtung umfasst alle für einen autonomen Betrieb erforderlichen Komponenten. Neben dem Okklusionsmittel, das zu intermittierenden Okklusion ansteuerbar ist und im Blutgefäß positioniert werden kann, umfasst die implantierbare Vorrichtung wenigstens einen Sensor sowie eine Steuereinrichtung, sodass das Okklusionsmittel in Abhängigkeit von den vom wenigstens einen Sensor erfassten Messwerten gesteuert werden kann. Dabei ist beispielsweise eine druckabhängige Steuerung möglich, wie in den Dokumenten US 4,934,996, WO 03/008018 A2, WO 2005/120602 A1 und WO 2005/120601 A1 beschrieben wurde. Die Steuerung umfasst hierbei insbesondere die Bestimmung der optimalen Zeitpunkte, zu welchen das Blutgefäß vom Okklusionsmittel verschlossen wird und diejenigen Zeitpunkte, zu welchen das Blutgefäß wiederum freigegeben wird. Die intermittierende Okklusion umfasst hierbei eine Mehrzahl von abwechselnd durchgeführten Okklusions- und Freigabephasen. Weiters umfasst die erfindungsgemäße Vorrichtung ein vom Okklusionsmittel gesondertes Verankerungsmittel, mit dessen Hilfe das Okklusionsmittel im Blutgefäß bzw. relativ zum Blutgefäß positioniert werden kann. Mit Hilfe des Verankerungsmittels kann eine dauerhafte, insbesondere axiale Fixierung des Okklusionsmittels und ggf. des wenigstens einen Sensors vorgenommen werden, sodass das Okklusionsmittel für lange Zeit im Blutgefäß verbleiben und vollkommen automatisch betrieben werden kann. Die Behandlung eines Patienten erfordert nur mehr einen einzigen operativen Eingriff durch den Arzt, nämlich das Implantieren der erfindungsgemäßen Vorrichtung in ein Blutgefäß des Patienten, wobei nicht nur das Okklusionsmittel implantiert wird, sondern auch der wenigstens eine Sensor, die Steuereinrichtung und das Verankerungsmittel. Nach der Implantierung kann der Patient das Krankenhaus verlassen, wobei die intermittierende Okklusion des Blutgefäßes je nach Bedarf vorgenommen wird. Dabei ist es denkbar, dass das Starten der intermittierenden Okklusion extern getriggert wird, beispielsweise vom Patienten selbst oder von einem Arzt oder dass aufgrund der vom wenigstens einen Sensor erfassten Messwerte eine selbsttätige Auslösung erfolgt.

Bei den intermittierenden Okklusionsvorrichtungen gemäß dem Stand der Technik wurde die intermittierende Okklusion mit Hilfe eines in das Blutgefäß eingeführten Ballons vorgenommen, der zur Okklusion des Blutgefäßes aufgedehnt und zum Aufheben der Okklusion wieder entleert wurde. Das Aufdehnen und Kontrahieren des Ballons erfolgte mit Hilfe eines gasförmigen oder flüssigen Mediums, das in den Ballon gepumpt und wiederum abgesaugt wurde. Ein derartiges Okklusionsmittel eignet sich nicht notwendiger Weise für das dauerhafte Implantieren, da zum einen ein entsprechendes Reservoir für das flüssige oder gasförmige Medium im Körper des Patienten vorgesehen werden müsste und andererseits das Risiko eines Platzens des Ballons und damit eines Austretens des Mediums zu hoch ist. Gemäß einer bevorzugten Weiterbildung der erfindungsgemäßen Vorrichtung ist das Okklusionsmittel daher nicht hydraulisch oder pneumatisch angetrieben, sondern als mechanisch oder elektrisch angetriebenes Okklusionsmittel ausgebildet, wobei der Antrieb bevorzugt elektrisch ansteuerbar ist. Ein derartiger mechanischer oder elektrischer Antrieb für das Okklusionsmittel kann mühelos implantiert werden, wobei für die elektrische Ansteuerung des Antriebs lediglich eine elektrische Energieversorgung vorzusehen ist. Die elektrische Energieversorgung kann hierbei ähnlich, wie dies auch bei Schrittmachern der Fall ist, an einer geeigneten Position im Körper des Patienten implantiert werden, wobei die elektrischen Verbindungsleitungen zum Okklusionsmittel bzw. zum Antrieb des Okklusionsmittels im Körper geführt sind. Die elektrische Energieversorgung kann hierbei beispielsweise mit der Steuereinrichtung zu einem einzigen Bauteil integriert werden, sodass der Aufwand für den operativen Eingriff verringert werden kann.

Mechanisch oder elektrisch angetriebene Okklusionsmittel sind in einer Vielzahl von Ausbildungen denkbar. Beispielsweise kann gemäß einer bevorzugten Ausbildung das Okklusionsmittel ein elektrisch ansteuerbares Ventil umfassen. Das Okklusionsmittel kann bevorzugt einen elektrisch ansteuerbaren Aktor aufweisen, der beispielsweise von einem Elektromagneten gebildet sein kann. Der Aktor kann mit wenigstens einem Bauteil zusammenwirken, der in Abhängigkeit von der Betätigung des Aktors bzw. des Elektromagneten zwischen einer das Blutgefäß okkludierenden Stellung und einer das Blutgefäß freigebenden Stellung bewegbar ist. Beispielsweise kann der Aktor mit einer faltbaren Membran zusammenwirken. Gemäß einer anderen Ausführungsform kann der Aktor mit einem nach Art eines Schirms aufspannbaren und zusammenklappbaren Verschlussgliedes zusammenwirken.

Denkbar ist auch eine Ausbildung, bei welcher der Aktor von einem ein Formgedächtnis aufweisenden Material ("shape memory material") gebildet ist. Die Form eines derartigen Materials ändert sich beispielsweise in Abhängigkeit von einer angelegten Spannung oder von der Temperatur. Auch elektroaktive Polymere sind in diesem Zusammenhang denkbar.

Bei einer anderen Ausbildung kann wenigstens eine Klappe beispielsweise aus leitendem Kunststoff vorgesehen sein, wobei sich die Klappe je nach elektrischem Ladungszustand von einem Gegenglied, beispielsweise einer weiteren Klappe abstoßen kann, wodurch die Klappe zwischen einer Schließ- und einer Öffnungsstellung bewegbar ist. Derartige Mechanismen sind beispielsweise im Zusammenhang mit Herzklappen bekannt geworden.

Bei denjenigen Ausbildungen, bei welchen das Okklusionsmittel wenigstens einen zwischen einer Schließstellung, in welcher das Blutgefäß okkludiert ist, und einer Öffnungsstellung, in welcher das Blutgefäß nicht okkludiert ist, bewegbaren Teil aufweist, sieht eine bevorzugte Weiterbildung vor, dass der Aktor bei elektrischer Ansteuerung mit dem bewegbaren Teil zur Aufbringung einer im Schließsinne wirkenden Kraft zusammenwirkt.

Dies hat zur Folge, dass der bewegbare Teil im Schließsinne beaufschlagt wird und der bewegbare Teil dementsprechend gegen den Widerstand des Blutflusses innerhalb des Blutgefäßes in die Schließstellung gebracht wird, sodass bei einem allfälligen Versagen des Aktors sich aufgrund des anstehenden Drucks des aufgestauten Blutes eine selbsttätige Rückstellung des bewegbaren Teils erfolgt, sodass das Blut in der Folge ungestört durch das Blutgefäß fließen kann. Dadurch wird die Betriebssicherheit der erfindungsgemäßen Vorrichtung wesentlich erhöht.

Um eine geeignete Verankerung des Okklusionsmittels zu erreichen, ist das Verankerungsmittel bevorzugt als Gefäßimplantat und insbesondere als in radialer Richtung aufweitbarer Stent ausgebildet. Derartige Stents sind in der Gefäßchirurgie allgemein bekannt und in der Regel derart ausgebildet, dass sie in komprimierter Form, in welcher sie einen reduzierten Außendurchmesser aufweisen, in das entsprechende Blutgefäß eingeführt werden können und nach Erreichen der gewünschten Position in eine expandierte Form, in welcher sie einen vergrößerten Außendurchmesser aufweisen, gebracht werden können. Im expandierten Zustand übt der Stent einen kontrollierten radialen Anpressdruck auf die Innenwand des Blutgefäßes aus, sodass die Fixierung der eingenommenen Position sichergestellt ist. Die Haltekraft kann durch eine spezielle Struktur des Stentmantels, wie beispielsweise durch eine Helixstruktur oder durch reibungserhöhende Maßnahmen am Außenmantel erhöht werden.

Wenigstens ein Teil des Aktors kann hierbei im Stent untergebracht sein.

Gemäß einer alternativen Ausbildung ist vorgesehen, dass das Verankerungsmittel von einem künstlichen Gefäß gebildet ist. Die Implantierung der erfindungsgemäßen Vorrichtung umfasst in diesem Fall das Ersetzen eines Abschnitts des Blutgefäßes mit dem künstlichen Gefäß, das mit dem angrenzenden Bereich des natürlichen Gefäßes verbunden und auf diese Art und Weise eine Verankerung des mit dem künstlichen Gefäß verbundenen Okklusionsmittel ermöglicht.

In bevorzugter Weise ist die Ausbildung derart weitergebildet, dass das Verankerungsmittel einen Aufnahmeraum für das Okklusionsmittel aufweist oder ausbildet. Das Okklusionsmittel ist in diesem Fall unmittelbar im Verankerungsmittel, wie beispielsweise im Stent oder im künstlichen Gefäß angeordnet, was den Vorteil mit sich bringt, dass das Implantat als vorgefertigte Einheit implantiert werden kann.

Wie bereits erwähnt, erfolgt die Steuerung des Okklusionsmittels in Abhängigkeit von wenigstens einem physiologischen Messwert. In diesem Zusammenhang ist die Ausbildung bevorzugt derart weitergebildet, dass ein Zeitglied vorgesehen ist, um eine Erfassung des wenigstens einen physiologischen Messwerts in vordefinierten Zeitabständen zu veranlassen. Je geringer die vordefinierten Zeitabstände sind, desto präziser kann die Steuerung erfolgen, da jeweils der aktuellste Messwert für die Steuerung herangezogen werden kann.

Bevorzugt ist ein Speicher für die physiologischen Messwerte und/oder die Okklusionsvorgänge vorgesehen, der mit einem Sensor zum drahtlosen Übermitteln des Speicherinhalts verbunden ist. Dabei kann beispielsweise ein externes Auswertegerät vorgesehen sein, das die im Speicher enthaltenen Daten drahtlos ausliest und dem Patienten und/oder dem behandelnden Arzt eine Analyse und Auswertung der Daten ermöglicht. Dadurch ist es in einfacher Art und Weise möglich, die Funktionsweise der implantierbaren Vorrichtung zu überprüfen und regelmäßige Diagnosen zu erstellen.

Die Steuerung der Okklusionsvorrichtung kann in unterschiedlichster Weise erfolgen. Bevorzugt ist vorgesehen, dass der Sensor zur Erfassung eines Flüssigkeitsdrucks, eines Flüssigkeitsvolumens, eines Flusses, eines elektrischen Widerstandes, einer elektrischen Impedanz, von Herzströmen zur Erstellung eines Elektrokardiogramms (EKG) und/oder eines metabolischen Parameters, wie z.B. der 02-Sättigung oder des pH-Werts bzw. des Laktatgehalts des Blutes, ausgebildet ist. Dabei kann lediglich einer dieser Parameter gemessen werden oder eine Mehrzahl. Üblicherweise müssen die entsprechenden Messwerte einer rechnerischen bzw. statistischen Auswertung zugeführt werden, um geeignete Steuersignale für das Okklusionsmittel zu generieren. Der Sensor kann dabei entweder im zu okkludierenden Blutgefäß, insbesondere an oder in der Nähe des Okklusionsmittels angeordnet sein oder auch in einer gesonderten, nicht im Blutgefäß angeordneten, aber ebenfalls implantierbaren Einheit, welche die Stromversorgung und/oder die Steuereinrichtung beherbergt. Wenn mehrere Sensoren vorgesehen sind, kann wenigstens ein Sensor im Blutgefäß und wenigstens ein anderer Sensor in der gesonderten Einheit angeordnet sein.

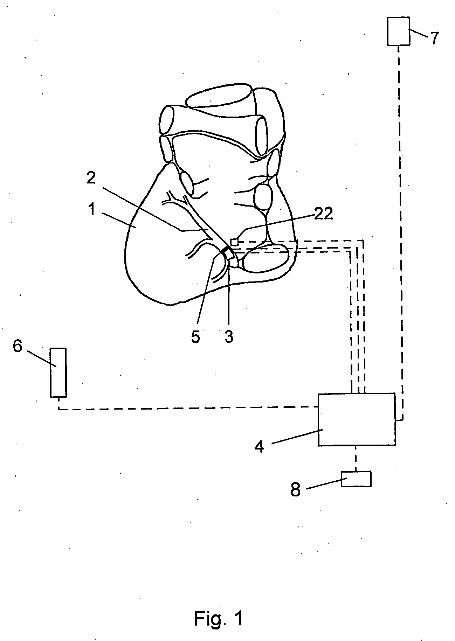

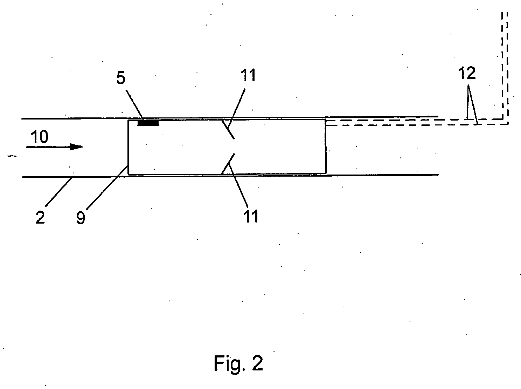

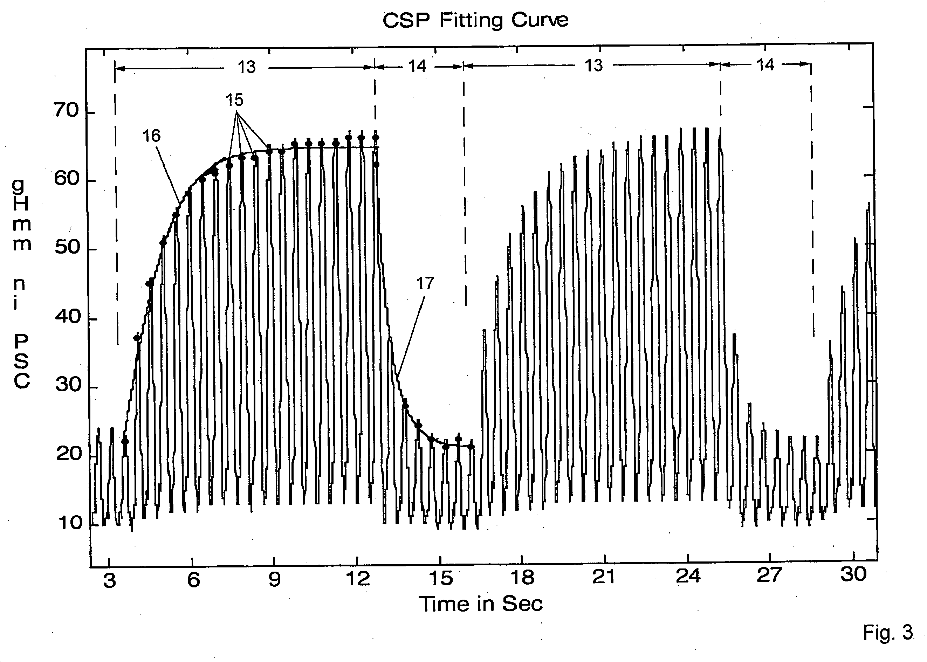

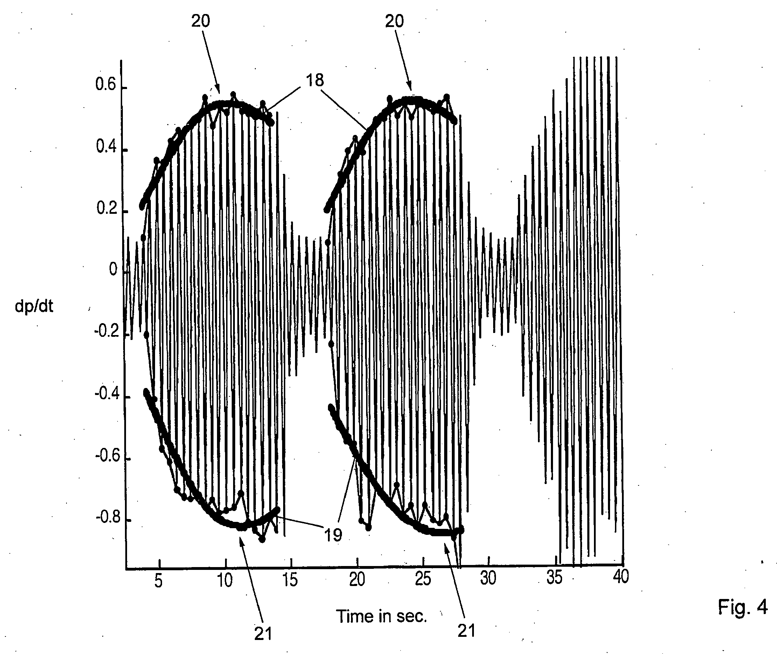

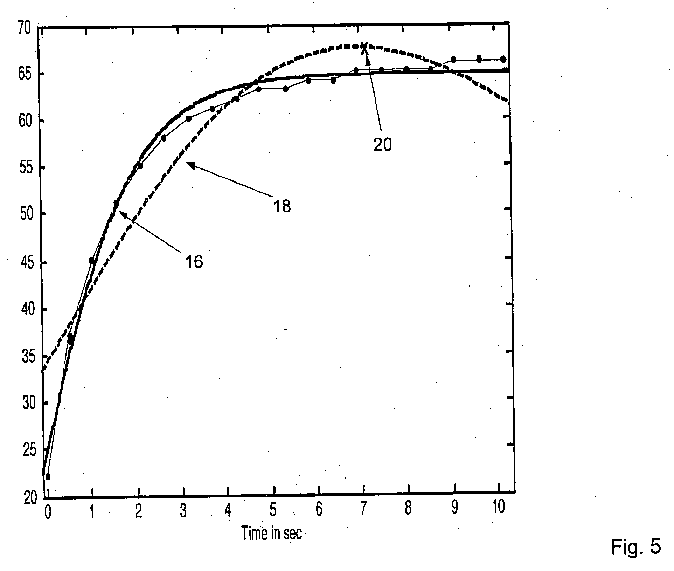

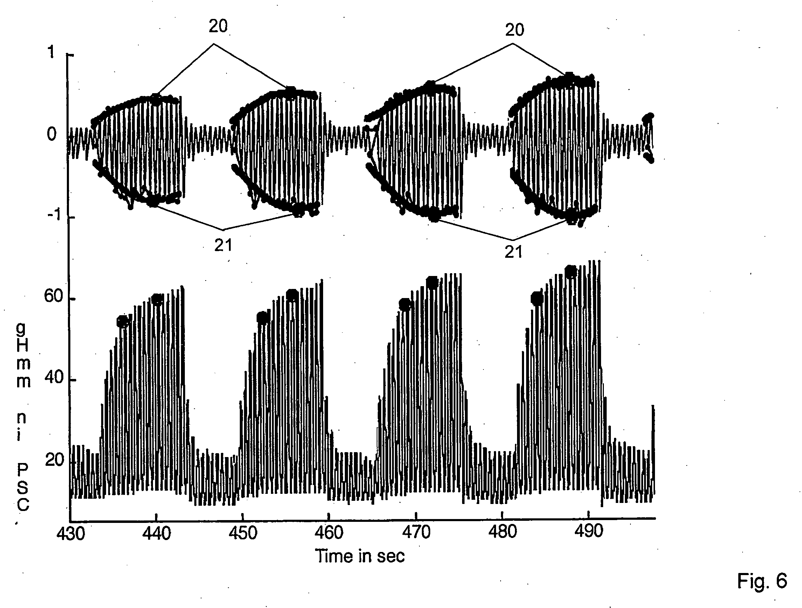

Die Erfindung wird nachfolgend anhand von in der Zeichnung schematisch dargestellten Ausführungsbeispielen näher erläutert. In dieser zeigen Fig.1 die erfindungsgemäße implantierbare Vorrichtung im implantierten Zustand, Fig.2 eine implantierte Okklusionsvorrichtung im Querschnitt, Fig.3 eine Darstellung der von einem Drucksensor gelieferten Druckkurve, Fig.4 die erste Ableitung der Druckkurve, Fig.5 die an die lokalen Maxima der Druckkurve und der ersten Ableitung angenäherten Kurven und Fig.6 die Kurven gemäß Fig. 3 und Fig.4 4 über eine Mehrzahl von Okklusions- und Freigabephasen.

In Fig.1 ist schematisch ein menschliches Herz 1 dargestellt. Im Koronarsinus 2 ist ein Okklusionsmittel 3 angeordnet, das zu intermittierender Okklusion des Koronarsinus 2 angesteuert werden kann. Die Steuereinrichtung für die Ansteuerung des Okklusionsmittels ist mit 4 bezeichnet. Der Steuervorrichtung 4 sind die Messwerte eines Sensors 5 über eine Leitung zugeführt. Der Sensor 5 ist beispielsweise als Blutdrucksensor ausgebildet und misst den Druck im Koronarsinus. An die Steuereinrichtung 4 ist weiters eine Elektrode 6 angeschlossen, über welche ein Stromimpuls emittiert werden kann. Mit Hilfe des Sensors 7, der in Bezug auf das Herz 1 bzw. die nicht dargstellte Lunge diametral gegenüber der Elektrode 6 angeordnet ist, kann die elektrische Impedanz des Thorax, bzw. des Herzens 1 und der Lunge ermittelt werden, die wiederum einen Rückschluss auf die Kontraktilität des Herzens 1 erlaubt. Weiters kann ein Sensor 22 vorgesehen sein, weicher der Messung der Leitfähigkeit des Blutes im Koronarsinus dient, wobei Studien ergeben haben, dass die Leitfähigkeit des Blutes ein Maß für die Kontraktilität des Herzens ist. In der Steuervorrichtung 4 werden die Messwerte der Sensoren 5 und 7 ausgewertet, wobei das Okklusionsmittel 3 in Abhängigkeit vom Ergebnis der Auswertung angesteuert wird. Insbesondere wird die Länge der einzelnen Okklusions- und Freigabephasen während der intermittierenden Okklusion bestimmt. Weiters wird auf Grund der Messwerte der optimale Zeitpunkt für den Beginn und die Beendigung der intermittierenden Okklusion bestimmt. Der genaue Steuerungsalgorithmus wird an Hand den Fig.3 bis 6 erläutert werden.

Die Stromversorgung der Steuereinrichtung 4 und ggf. des Okklusionsmittels 3 ist mit 8 bezeichnet.

In Fig.2 ist der Koronarsinus näher dargestellt und es ist ersichtlich, dass in den Koronarsinus 2 ein als Stent ausgebildetes Verankerungsmittel 9 eingeschoben wurde. Der Stent 9 wirkt mit der Innenwand des Koronarsinus 2 zusammen, sodass der Stent 9 in seiner Position fixiert ist. Die Flussrichtung des Blutes bei geöffnetem Okklusionsmittel 3 ist mit 10 bezeichnet. Der Stent 9 trägt einen Drucksensor 5, der den Druck im Koronarsinus 2 misst. Das Okklusionsmittel wird von wenigstens zwei klappenartigen Bauteilen 11 gebildet, die zwischen einer Schließstellung, in welcher der Koronarsinus 2 okkludiert ist, und einer Öffnungsstellung, in welcher der Koronarsinus geöffnet, d.h. nicht okkludiert ist, bewegt werden können. Die Bewegung der klappenartigen Bauteile kann durch Verschwenkung erfolgen. In Fig.2 sind die klappenartigen Bauteile 11 in einer Zwischenstellung gezeigt. Zum Okkludieren werden die klappenartigen Bauteile 11 entgegen der Richtung des Pfeils 10 in die Schließstellung verschwenkt und durch Kraftbeaufschlagung in dieser Stellung gehalten. Sobald die Kraftbeaufschlagung beendet wird, d.h. sobald die Ansteuerung des Okklusionsmittels beendet wird, werden die klappenartigen Bauteile 11 durch den anstehenden Blutdruck selbsttätig aufgedrückt, sodass eine automatische Öffnung des Koronarsinus bewirkt wird. Eine derartige Konfiguration hat zur Folge, dass der Koronarsinus 2 auch bei einer allfälligen Fehlfunktion, beispielsweise bei einem Versiegen der Stromquelle, selbsttätig geöffnet wird, sodass die Fehlfunktion nicht zu Beschädigungen des Organismus führen kann.

Wenn das Okklusionsmittel den Koronarsinus 2 während einer Okklusionsphase verschließt, staut sich das Blut im Koronarsinus 2 auf und retroperfundiert in das umliegende Gewebe. Bei Öffnen des Okklusionsmittels während der darauf folgenden Freigabephase wird das aufgestaute Blut ausgeschwemmt. Die abwechselnden Okklusions- und Freigabephasen wiederholen sich solange, bis auf Grund der Messwerte eine Verbesserung der Situation festgestellt wird.

Der Stent 9 ist mit zwei elektrischen Leitungen 12 verbunden, wobei die eine Leitung der Übertragung der Messwerte des Sensors 5 an die Steuereinrichtung 4 und die andere Leitung 12 der elektrischen Ansteuerung des Okklusionsmittels dient.

Fig. 3 zeigt die vom Drucksensor 5 ermittelten Messwerte. Die Druckkurve gibt den Verlauf des Drucks in mm Hg über die Zeit wieder. Es ist ersichtlich, dass das während jeden Herzschlages auftretende Druckmaximum während der Okklusionsphase 13 mit jedem Herzschlag ansteigt, bis die Druckmaxima einen oberen Plateauwert erreichen, wobei die Druckmaxima mit 15 bezeichnet sind. Während der Freigabephase 14 sinkt der Druck schlagartig ab, wobei die Druckmaxima einen unteren Plateauwert erreichen. Die Druckmaxima 15 bilden hierbei eine Extremwertreihe. Die Druckmaxima 15 können hierbei durch eine Kurve 16 (Okklusionsphase) und eine Kurve 17 (Freigabephase) angenähert werden. Die Annäherung erfolgt hierbei bevorzugt nach der Methode der kleinsten Fehlerquadrate. Die Wahl der zeitlichen Dauer der einzelnen Okklusions- und Freigabephasen kann auf Grundlage der angenäherten Kurven 16, 17 erfolgen.

Die Wahl der Dauer der einzelnen Okklusions- und Freigabephasen kann aber auch auf Grundlage der zeitlichen Ableitung dp/dt der Druckkurve erfolgen. Die erste Ableitung dp/dt der Druckkurve ist in Fig.4 dargestellt. Die Kurve zeigt, dass während jedes Herzschlages wiederum ein lokales Maximum und ein lokales Minimum der ersten Ableitung auftreten. Die Stelle des lokalen Maximums gibt hierbei den Zeitpunkt an, zu dem der Druckanstieg am schnellsten erfolgt. Die Stelle des lokalen Minimums gibt den Zeitpunkt an, zu welchem der Druckabfall am schnellsten erfolgt. In Fig. 4 sind für die Okklusionsphase die an die lokalen Maxima der ersten Ableitung angenäherte Kurve 18 und die an die lokalen Minima der ersten Ableitung angenäherte Kurve 19 dargestellt. Es ist ersichtlich, dass die bei aufeinander folgenden Herzschlägen auftretenden Maxima der ersten Ableitung zunächst höher werden, bis sie an dem Punkt 20 während der Okklusionsphase das Maximum erreichen und danach wieder sinken. Dasselbe gilt für den Punkt 21 der bei aufeinander folgenden Herzschlägen auftretenden Minima der ersten Ableitung. Die Punkte 20 und 21 können rechnerisch ermittelt werden, indem die Ableitung der angenäherten Kurven 18 und 19 gleich Null gesetzt werden.

Die positiven Werte der zeitlichen Ableitung dp/dt der Druckkurve (vgl. Kurve 18) sind ein Indikator für die Kontraktilität des Herzens, wobei die Okklusionsphase 13 beendet wird, wenn die Kontraktilität ein Maximum erreicht. Die Kontraktilität des Herzens kann aber nicht nur an Hand der zeitlichen Ableitung dp/dt der Druckkurve ermittelt werden, sondern stattdessen auf Grundlage der, mit dem Sensor 22 ermittelten Leitfähigkeit des Blutes im Koronarsinus.

Bei der Darstellung gemäß Fig. 5 sind die an die lokalen Maxima der Druckkurve angenäherte Kurve 16 und die an die lokalen Maxima der ersten Ableitung dp/dt der Druckkurve angenäherte Kurve 18 übereinander dargestellt. Es ist ersichtlich, dass das Maximum 20 der Kurve 18 einen wesentlich besser erkennbaren und eindeutigeren Anhaltspunkt zur Beendigung der Okklusionsphase bietet als der Plateauwert der Kurve 16.

Auch die negativen Werte der zeitlichen Ableitung dp/dt können herangezogen werden, um den optimalen Zeitpunkt der Beendigung der jeweiligen Okklusionsphase zu bestimmen. Die negativen Werte der zeitlichen Ableitung dp/dt sind ein Indikator für die Relaxationsphase des Herzens. Der Zeitpunkt der maximalen Druckabfallsgeschwindigkeit (der Zeitpunkt des jeweils während jedes Herzschlages auftretenden lokalen Minimums der erstens Ableitung) stellt nämlich den Beginn der isovolumetrischen Relaxationsphase des Herzens dar. Wenn die Okklusionsphase nun zu lange dauert, verkürzt sich die isovolumetrische Relaxationsphase. Um diese Verkürzung zu vermeiden, muss die Okklusionsphase rechtzeitig beendet werden. Dies gelingt durch die ständige Auswertung der ersten Ableitung der Druckkurve hinsichtlich der lokalen Minima, wobei in Kombination mit der Ermittlung der Herzströme (EKG) die Dauer der Relaxationsphase errechnet und eine Trenderkennung durchgeführt werden kann.

In Fig. 6 sind die Druckkurve und die entsprechende erste Ableitung dp/dt für eine Mehrzahl aufeinander abfolgender Okklusion- und Freigabephasen dargestellt. Ebenso sind die angenäherten Kurven 18 und 19 dargestellt und das während der einzelnen Okklusionsphasen jeweils auftretende Maximum 20 und das Minimum 21 eingezeichnet. Aus den Maxima 20 und den Minima 21 kann nun jeweils eine Extremwertreihe gebildet werden, die jeweils hinsichtlich der Entwicklung des Maximums bzw. Minimums ausgewertet werden kann, um auf Grundlage des Ergebnisses dieser Auswertung den optimalen Zeitpunkt der Beendigung der intermittierenden Okklusion zu bestimmen. Bei einem erfolgreichen Verlauf der Behandlung mittels intermittierender Okklusion kann der Wert des Punktes 20 von einer Okklusionsphase zur nächsten Okklusionsphase steigen oder über eine Mehrzahl von Okklusionsphasen hinweg wenigstens ein Trend in Richtung einer Steigerung des Werts erkennbar sein. Die Steigung des Werts des Punkts 20 lässt auf eine Steigerung der myokaridialen Kontraktilität schließen. Wenn der Wert des Punktes 20 nun einen vorgegebenen Sollwert oder einen Plateauwert erreicht, kann die intermittierende Okklusion und damit die Behandlung beendet werden.

Häufiger wird aber während einer Behandlung mittels intermittierender Okklusion beobachtet, dass der Wert des Punktes 21 niedriger wird. Der Wert des Punktes 21 ist hierbei repräsentativ für die diastolische Phase der Relaxation des Herzens. Der Wert ist umso niedriger, je größer die linksventrikuläre Druckabfallgeschwindigkeit ist, d.h. je schneller der Druck im okkludierten Koronarsinus während der Diastole absinkt. Die Bestimmung des Zeitpunkts des Endes der intermittierenden Okklusion kann also auch in Abhängigkeit des Werts des Punktes 21 erfolgen. Die intermittierende Okklusion und damit die Behandlung können beispielsweise dann beendet werden, wenn der Wert des Punktes 21 einen vorgegebenen Sollwert oder einen Plateauwert erreicht.

Auch eine Kombination der Auswertung der Änderung des Werts des Punktes 20 und der Änderung des Werts des Punktes 21 kann zielführend sein.

Eine weitere Behandlung kann in der Folge wieder erforderlich werden, wenn die ständige Überwachung von definierten physiologischen Parametern ergibt, dass sich die Leistung des Herzens verschlechtert, etwa wenn eine Messung der elektrischen Impedanz oder der elektrischen Leitfähigkeit des Thorax, insbesondere des Herzens und/oder der Lunge, ein Ansteigen des Flüssigkeitsgehalts in diesem Bereich erkennen lässt.

## Patentansprüche

1. Vorrichtung zur intermittierenden Okklusion eines Blutgefäßes, insbesondere einer das Organsystem drainierenden Vene, umfassend
- ein zu intermittierender Okklusion ansteuerbares Okklusionsmittel (3),
- eine mit dem Okklusionsmittel (3) verbundene Steuereinrichtung (4),
- wenigstens einen Sensor (5,6,7) zur kontinuierlichen oder in Zeitabständen vorgenommenen Erfassung wenigstens eines physiologischen Messwerts und
- einen vom Sensor gespeisten Messwertspeicher zum Speichern einer Messwertreihe,
**dadurch gekennzeichnet, dass** die Messwertreihe der Steuereinrichtung (4) zuführbar ist, die zur rechnerischen, insbesondere statistischen Auswertung der Messwertreihe ausgebildet ist und mit der Okklusionseinrichtung (3) zum Beginnen oder zur Beendigung des Vorgangs der intermittierenden Okklusion, bei welchem das Blutgefäß abwechselnd okkludiert und freigegeben wird, in Abhängigkeit vom Ergebnis der Auswertung zusammenwirkt.

2. Vorrichtung nach Anspruch 1, **dadurch gekennzeichnet, dass** die Steuereinrichtung (4) eine Triggerschaltung zum Auslösen der rechnerischen Auswertung nach jeder Messwertermittlung aufweist.

3. Vorrichtung nach Anspruch 1 oder 2, **dadurch gekennzeichnet, dass** die Steuereinrichtung (4) eine Umrechenschaltung zur Umrechnung der einzelnen Messwerte der Messwertreihe in abgeleitete Messwerte aufweist, wobei eine abgeleitete Messwertreihe erhalten wird.

4. Vorrichtung nach Anspruch 1, 2 oder 3, **dadurch gekennzeichnet, dass** die Steuereinrichtung (4) eine Differenzwertermittlungsschaltung zur Ermittlung der Differenz der jeweils letzten zwei Messwerte der Messwertreihe oder der Differenz der jeweils letzten zwei abgeleiteten Messwerte der abgeleiteten Messwertreihe aufweist, wobei eine Differenzwertreihe erhalten wird.

5. Vorrichtung nach Anspruch 4, **dadurch gekennzeichnet, dass** die Differenzwertermittlungsschaltung zur Ermittlung der kumulierten Differenzen der Differenzwertreihe ausgebildet ist.

6. Vorrichtung nach einem der Ansprüche 1 bis 5, **dadurch gekennzeichnet, dass** die Steuereinrichtung (4) eine Messwertveränderungsermittlungsschaltung zur Ermittlung der Änderung der Messwerte der Messwertreihe oder der abgeleiteten Messwerte der abgeleiteten Messwertreihe je Zeiteinheit aufweist, wobei eine Ableitungsreihe erhalten wird.

7. Vorrichtung nach einem der Ansprüche 1 bis 6, **dadurch gekennzeichnet, dass** die Steuereinrichtung (4) eine Extremwertermittlungsschaltung zur Ermittlung von lokalen Maxima (15) und/oder lokalen Minima der Werte der Messwertreihe, der umgewandelten Messwertreihe, der Differenzwertreihe und/oder der Ableitungsreihe und zur Bildung einer Extremwertreihe (16,17,18,19) aus den lokalen Maxima (15) bzw. Minima aufweist.

8. Vorrichtung nach Anspruch 7, **dadurch gekennzeichnet, dass** die Extremwertexmittlungsschaltung derart ausgebildet ist, dass als lokales Maximum bzw. Minimum das jeweils während einer Okklusion auftretende Maximum (20) bzw. Minimum (21) der genannten Werte gewählt wird.

9. Vorrichtung nach einem der Ansprüche 1 bis 8, **dadurch gekennzeichnet, dass** die Steuereinrichtung (4) eine Vergleichsschaltung zum Vergleichen der Werte der Messwertreihe, der abgeleiteten Messwertreihe, der Differenzwertreihe, der Ableitungsreihe und/oder der Extremwertreihe und/oder der kumulierten Differenzen mit einem vorgegebenen Grenzwert aufweist und mit dem Okklusionsmittel derart zusammenwirkt, dass die intermittierende Okklusion bei Erreichen des Grenzwerts begonnen wird.

10. Vorrichtung nach einem der Ansprüche 1 bis 9, **dadurch gekennzeichnet, dass** die Steuereinrichtung (4) eine Auswerteschaltung zur Erkennung oder Abschätzung eines Plateauwerts der Werte der Messwertreihe, der abgeleiteten Messwertreihe, der Differenzwertreihe, der Ableitungsreihe und/oder der Extremwertreihe aufweist, die derart mit dem Okklusionsmittel zusammenwirkt, dass die intermittierende Okklusion beim Plateauwert oder bei einem vorbestimmten Prozentwert des Plateauwerts beendet wird.

11. Vorrichtung nach einem der Ansprüche 1 bis 10, **dadurch gekennzeichnet, dass** die Steuereinrichtung (4) mit dem Okklusionsmittel derart zusammenwirkt, dass beim Beginnen und/oder beim Beenden der intermittierenden Okklusion jeweils eine neue Messwertreihe begonnen wird.

12. Vorrichtung nach einem der Ansprüche 1 bis 11, **dadurch gekennzeichnet, dass** der Sensor (5,6,7) zur Erfassung des Blutdrucks im Bereich des Blutgefäßes (2), des Blutmassen- oder -volumenflusses im Bereich des Blutgefäßes (2), des elektrischen Widerstands, der Leitfähigkeit, der elektrischen Impedanz im Bereich des Blutgefäßes (2), insbesondere des Herzens (1) und/oder der Lunge, des EKG und/oder eines metabolischen Parameters, wie z.B. der O2-Sättigung und/oder des Laktatgehalt bzw. des pH-Werts des Blutes, ausgebildet ist.

13. Vorrichtung nach einem der Ansprüche 1 bis 12, **dadurch gekennzeichnet, dass** der Sensor eine Elektrode (6) zum Aussenden eines Stromimpulses umfasst.

14. Vorrichtung nach einem der Ansprüche 1 bis 13, **dadurch gekennzeichnet, dass** eine Auswerteschaltung zum Auswerten eines zugeführten EKGs, insbesondere zur Erkennung einer ST-Hebung, vorgesehen ist.

15. Vorrichtung nach einem der Ansprüche 1 bis 14, **dadurch gekennzeichnet, dass** die Steuereinrichtung (4) eine Auswerteschaltung zur jeweils gesonderten rechnerischen Auswertung, insbesondere nach Art wenigstens eines der Ansprüche 3 bis 7, der während der Okklusion des Blutgefäßes (2) ermittelten Messwerten aufweist, die mit dem Okklusionsmittel (3) derart zusammenwirkt, dass die Okklusion in Abhängigkeit vom Ergebnis der Auswertung beendet wird.

16. Vorrichtung nach einem der Ansprüche 1 bis 15, **dadurch gekennzeichnet, dass** die Steuereinrichtung (4) eine Auswerteschaltung zur jeweils gesonderten rechnerischen Auswertung, insbesondere nach Art wenigstens eines der Ansprüche 3 bis 7, der wahrend der Freigabe des Blutgefäßes (2) ermittelten Messwerte aufweist, die mit dem Okklusionsmittel (3) derart zusammenwirkt, dass die Okklusion in Abhängigkeit vom Ergebnis der Auswertung eingeleitet wird.

17. Vorrichtung nach einem der Ansprüche 1 bis 16, **dadurch gekennzeichnet, dass** der Sensor als Blutdrucksensor (5) ausgebildet und im okkludierten Blutgefäß positionierbar ist, wobei die Steuereinrichtung mit dem Okklusionsmittel derart zusammenwirkt, dass die Okklusion bei Erreichen eines Grenzwerts aufgehoben wird.

18. Vorrichtung nach einem der Ansprüche 1 bis 17, **dadurch gekennzeichnet, dass** das Blutgefäß (2) der Koronarsinus ist.

19. Vorrichtung nach einem der Ansprüche 1 bis 18, **dadurch gekennzeichnet, dass** die Vorrichtung als implantierbare Vorrichtung ausgebildet ist.

## Claims

1. A device for the intermittent occlusion of a blood vessel, in particular a vein draining the organ system, comprising
- an occlusion means (3) capable of being controlled for intermittent occlusion,
- a control device (4) connected to the occlusion means (3),
- at least one sensor (5,6,7) for continuously or intermittently detecting at least one physiological measurement value, and
- a data storage for storing a series of measurement values, which is fed by the sensor,
**characterized in that** the series of measurement values can be fed to the control device (4), which is arranged for the computational, in particular statistical, evaluation of the series of measurement values and cooperates with the occlusion means (3) for initiating or terminating the process of intermittent occlusion, during which the blood vessel is alternately occluded and released as a function of the result of the evaluation.

2. A device according to claim 1, **characterized in that** the control device (4) comprises a trigger circuit for triggering the computational evaluation after each determination of a measurement value.

3. A device according to claim 1 or 2, **characterized in that** the control device (4) comprises a conversion circuit for converting the individual measurement values of the series of measurement values into derived measurement values, thus obtaining a series of derived measurement values.

4. A device according to claim 1, 2 or 3, **characterized in that** the control device (4) comprises a difference value determination circuit for determining the difference of the respectively last two measurement values of the series of measurement values, or the difference of the respectively last two derived measurement values of the series of derived measurement values, thus obtaining a series of difference values.

5. A device according to claim 4, **characterized in that** the difference value determination circuit is arranged to determine the cumulated differences of the series of difference values.

6. A device according to any one of claims 1 to 5, **characterized in that** the control device (4) comprises a measurement value change determination circuit for determining the changes in the measurement values of the series of measurement values, or the derived measurement values of the series of derived measurement values, per unit time, thus obtaining a derivation series.

7. A device according to any one of claims 1 to 6, **characterized in that** the control device (4) comprises an extreme value determination circuit for determining local maxima (15) and/or local minima of the values of the series of measurement values, the series of converted measurement values, the series of difference values and/or the derivation series, and for forming a series of extreme values (16,17,18,19) from the local maxima (15) and minima, respectively.

8. A device according to claim 7, **characterized in that** the extreme value determination circuit is configured such that the maximum (20) or minimum (21) respectively occurring during an occlusion, of the said values is selected as the local maximum or minimum, respectively.

9. A device according to any one of claims 1 to 8, **characterized in that** the control device (4) comprises a comparator circuit for comparing the values of the series of measurement values, the series of derived measurement values, the series of difference values, the derivation series and/or the series of extreme values and/or the cumulated differences to a pregiven limit value, and cooperates with the occlusion means in such a manner that the intermittent occlusion is initiated when the limit value is reached.

10. A device according to any one of claims 1 to 9, **characterized in that** the control device (4) comprises an evaluation circuit for identifying or estimating a plateau value of the values of the series of measurement values, the series of derived measurement values, the series of difference values, the derivation series and/or the series of extreme values, which evaluation circuit cooperates with the occlusion means in such a manner that the intermittent occlusion is terminated at the plateau value, or at a predetermined percentage of the plateau value.

11. A device according to any one of claims 1 to 10, **characterized in that** the control device (4) cooperates with the occlusion means in such a manner that a new series of measurement values is each started when the intermittent occlusion is initiated and/or terminated.

12. A device according to any one of claims 1 to 11, **characterized in that** the sensor (5,6,7) is arranged to detect the blood pressure within the blood vessel (2), the blood mass or volume flow rate within the blood vessel (2), the electrical resistance, the conductivity, the electrical impedance within the blood vessel (2), in particular the heart (1) and/or the lung, the ECG and/or a metabolic parameter such as the oxygen saturation and/or the lactate level or the pH of the blood.

13. A device according to any one of claims 1 to 12, **characterized in that** the sensor comprises an electrode (6) for emitting a current impulse.

14. A device according to any one of claims 1 to 13, **characterized in that** an evaluation circuit for evaluating a fed ECG and, in particular, detecting an ST elevation is provided.

15. A device according to any one of claims 1 to 14, **characterized in that** the control device (4) comprises an evaluation circuit for the respectively separate computational evaluation, in particular in the manner of at least one of claims 3 to 7, of the measurement values determined during the occlusion of the blood vessel (2), which evaluation circuit cooperates with the occlusion means (3) in such a manner that the occlusion is terminated as a function of the result of the evaluation.

16. A device according to any one of claims 1 to 15, **characterized in that** the control device (4) comprises an evaluation circuit for the respectively separate computational evaluation, in particular in the manner of at least one of claims 3 to 7, of the measurement values determined during the release of the blood vessel (2), which evaluation circuit cooperates with the occlusion means (3) in such a manner that the occlusion is initiated as a function of the result of the evaluation.

17. A device according to any one of claims 1 to 16, **characterized in that** the sensor is configured as a blood pressure sensor (5) and positionable in the occluded blood vessel, wherein the control device cooperates with the occlusion means in such a manner that the occlusion is released when a limit value is reached.

18. A device according to any one of claims 1 to 17, **characterized in that** the blood vessel (2) is the coronary sinus.

19. A device according to any one of claims 1 to 18, **characterized in that** the device is designed as an implantable device.

## Revendications

1. Dispositif destiné à l'occlusion intermittente d'un vaisseau sanguin, en particulier d'une veine drainant le système d'organes, comprenant
- un moyen d'occlusion (3) pouvant être amorcé pour créer une occlusion intermittente,
- un dispositif de commande (4) relié au moyen d'occlusion (3),
- au moins un capteur (5, 6, 7) pour la détection en continu ou réalisée à des intervalles de temps d'au moins une valeur de mesure physiologique et
- une mémoire de valeurs de mesure alimentée par le capteur pour le stockage d'une série de valeurs de mesure,
**caractérisé en ce que** la série de valeurs de mesure peut être amenée au dispositif de commande (4) qui est réalisé pour une évaluation mathématique, en particulier statistique, de la série de valeurs de mesure et coopère avec le dispositif d'occlusion (3) au début ou à la fin du processus d'occlusion intermittente, dans lequel le vaisseau sanguin est alternativement occlus et ouvert, en fonction du résultat de l'évaluation.

2. Dispositif selon la revendication 1, **caractérisé en ce que** le dispositif de commande (4) comporte un circuit à déclenchement pour déclencher l'évaluation mathématique après chaque détermination de valeur de mesure.

3. Dispositif selon la revendication 1 ou 2, **caractérisé en ce que** le dispositif de commande (4) comporte un circuit de conversion pour convertir les valeurs de mesure individuelles de la série de valeurs de mesure en valeurs de mesure dérivées, dans lequel une série de valeurs de mesure dérivées est obtenue,

4. Dispositif selon la revendication 1, 2 ou 3, **caractérisé en ce que** le dispositif de commande (4) comporte un circuit de détermination de valeurs différentielles pour déterminer la différence des respectivement deux dernières valeurs de mesure de la série de valeurs de mesure ou la différence des respectivement deux dernières valeurs de mesure dérivées de la série de valeurs de mesure dérivées, dans lequel une série de valeurs différentielles est obtenue.

5. Dispositif selon la revendication 4, **caractérisé en ce que** le circuit de détermination de valeurs différentielles est réalisé pour déterminer les différences cumulées de la série de valeurs différentielles.

6. Dispositif selon l'une quelconque des revendications 1 à 5, **caractérisé en ce que** le dispositif de commande (4) comporte un circuit de détermination de modification des valeurs de mesure pour déterminer la modification des valeurs de mesure de la série de valeurs de mesure ou des valeurs de mesure dérivées de la série de valeurs de mesure dérivées par unité de temps, dans lequel une série de dérivation est obtenue.

7. Dispositif selon l'une quelconque des revendications 1 à 6, **caractérisé en ce que** le dispositif de commande (4) comporte un circuit de détermination de valeurs extrêmes pour déterminer des maximas locaux (15) et/ou des minimas locaux des valeurs de la série de valeurs de mesure, de la série de valeurs de mesure converties, de la série de valeurs différentielles et/ou de la série de dérivation et pour former une série de valeurs extrêmes (16, 17, 18, 19) à partir des maximas (15) ou des minimas locaux.

8. Dispositif selon la revendication 7, **caractérisé en ce que** le circuit de détermination de valeurs extrêmes est réalisé de telle sorte qu'est choisi comme maximum ou minimum local le maximum (20) ou le minimum (21) des valeurs mentionnées apparaissant respectivement pendant une occlusion.

9. Dispositif selon l'une quelconque des revendications 1 à 8, **caractérisé en ce que** le dispositif de commande (4) comporte un circuit de comparaison pour comparer les valeurs de la série de valeurs de mesure, de la série de valeurs de mesure dérivées, de la série de valeurs différentielles, de la série de dérivation et/ou de la série de valeurs extrêmes et/ou des différences cumulées à une valeur limite prédéfinie et coopère avec le moyen d'occlusion de telle sorte que l'occlusion intermittente débute à l'atteinte de la valeur limite.

10. Dispositif selon l'une quelconque des revendications 1 à 9, **caractérisé en ce que** le dispositif de commande (4) comporte un circuit d'évaluation pour identifier ou estimer une valeur de plateau des valeurs de la série de valeurs de mesure, de la série de valeurs de mesure dérivées, de la série de valeurs différentielles, de la série de dérivation et/ou de la série de valeurs extrêmes, qui coopère avec le moyen d'occlusion de telle sorte que l'occlusion intermittente prend fin à la valeur plateau ou à une valeur de pourcentage prédéterminée de la valeur plateau.

11. Dispositif selon l'une quelconque des revendications 1 à 10, **caractérisé en ce que** le dispositif de commande (4) coopère avec le moyen d'occlusion de telle sorte qu'une nouvelle série de valeurs de mesure débute respectivement au début et/ou à la fin de l'occlusion intermittente.

12. Dispositif selon l'une quelconque des revendications 1 à 11, **caractérisé en ce que** le capteur (5,6,7) est réalisé pour détecter la pression sanguine dans la zone du vaisseau sanguin (2), le débit massique ou débit volumétrique sanguin dans la zone du vaisseau sanguin (2), la résistance électrique, la conductivité, l'impédance électrique dans la zone du vaisseau sanguin (2), en particulier du coeur (1) et/ou du poumon, l'ECG et/ou un paramètre métabolique, tel que par exemple la saturation en 02 et/ou la teneur en lactate ou la valeur de pH du sang.

13. Dispositif selon l'une quelconque des revendications 1 à 12, **caractérisé en ce que** le capteur comprend une électrode (6) pour émettre une impulsion de courant.

14. Dispositif selon l'une quelconque des revendications 1 à 13, **caractérisé en ce qu'**est prévu un circuit d'évaluation pour évaluer un ECG mis à disposition, en particulier pour la détection d'une élévation du ST.

15. Dispositif selon l'une quelconque des revendications 1 à 14, **caractérisé en ce que** le dispositif de commande (4) comporte un circuit d'évaluation pour l'évaluation mathématique respectivement séparée, en particulier à la manière d'au moins une des revendications 3 à 7, des valeurs de mesure déterminées pendant l'occlusion du vaisseau sanguin (2), qui coopère avec le moyen d'occlusion (3) de telle sorte qu'il est mis fin à l'occlusion en fonction du résultat de l'évaluation.

16. Dispositif selon l'une quelconque des revendications 1 à 15, **caractérisé en ce que** le dispositif de commande (4) comporte un circuit d'évaluation pour l'évaluation mathématique respectivement séparée, en particulier à la manière d'au moins une des revendications 3 à 7, des valeurs de mesure déterminées pendant l'ouverture du vaisseau sanguin (2), qui coopère avec le moyen d'occlusion (3) de telle sorte que l'occlusion est commencée en fonction du résultat de l'évaluation.

17. Dispositif selon l'une quelconque des revendications 1 à 16, **caractérisé en ce que** le capteur est réalisé sous la forme d'un capteur de pression sanguine (5) et positionnable dans le vaisseau sanguin occlus, dans lequel le dispositif de commande coopère avec le moyen d'occlusion de telle sorte que l'occlusion est débloquée à l'atteinte d'une valeur limite.

18. Dispositif selon l'une quelconque des revendications 1 à 17, **caractérisé en ce que** le vaisseau sanguin (2) est le sinus coronaire.

19. Dispositif selon l'une quelconque des revendications 1 à 18, **caractérisé en ce que** le dispositif est réalisé sous la forme d'un dispositif implantable.
